# EUROPEAN PATENT APPLICATION

(11) **EP 4 799 635 A1**
(43) Date of publication of application: **02.09.2026**
(21) Application number: 24881334.7
(22) Date of filing: 23.09.2024
(51) Int. Cl.: A61K 38/17, C07K 14/47, A61P 11/00

(54) **DRUG FOR TREATING PULMONARY FIBROSIS**

(30) Priority: 25.10.2023 CN 202311392413
(71) Applicant: Shanghai Seme Cell Technology Co., Ltd, Shanghai 200241 (CN)
(72) Inventor: YAO, Liudi, Shanghai 200241 (CN)
(74) Representative: Mewburn Ellis LLP
(86) International application number: PCT/CN2024/120465
(87) International publication number: WO 2025/086968

(57) **Abstract**

The present invention relates to a drug for preventing and/or treating idiopathic pulmonary fibrosis, and specifically provides use of annexin A5 in the preparation of a composition for preventing and/or treating idiopathic pulmonary fibrosis. Annexin A5 can effectively treat/slow down idiopathic pulmonary fibrosis, reduce the severity of pulmonary interstitial lesions, inhibit the differentiation of fibroblasts and the deposition of collagen, decrease the release of pro-inflammatory factors, and maintain the normal morphology of alveolar epithelium.

## Description

### TECHNICAL FIELD

The present invention relates to the field of protein drugs, and more particularly to use of annexin A5 in the treatment of idiopathic pulmonary fibrosis.

### BACKGROUND

Fibrosis is a hallmark of many chronic degenerative diseases and a majoy cause of death in the current population. There are over 3 million people with idiopathic pulmonary fibrosis (IPF) globally, whose incidence is similar to that of gastric cancer and brain cancer, and its incidence has increased exponentially over the past 20 years. Studies show that middle-aged and elderly men are the primary affected population. Early symptoms of IPF are insidious, and as scars accumulate, their symptoms often take years or even decades to gradually appear. Common symptoms include shortness of breath, dry cough, fatigue, weight loss, and clubbing of the fingers. Due to patient heterogeneity and the lack of specificity in symptoms, current diagnosis of IPF requires a combined diagnosis of chest HRCT and lung histopathology to exclude other interstitial lung diseases, which usually takes 1-2 years. This delayed diagnosis significantly impacts the survival of IPF patients, with a median survival of only 2-3 years. In China, there are currently no publicly available large-scale epidemiological surveys on the incidence of IPF, but due to the large population and aging trend, the burden of IPF on individuals, families, society and public health resources in China cannot be underestimated.

Treatment options for IPF are limited. Due to the irreversible lung function damage, lung transplantation remains the only effective treatment method that can prolong patient survival. According to incomplete statistics, at least dozens of drugs for treating IPF are currently under development, with a few of them already entering Phase II and Phase III clinical trials, but their efficacy and safety still need to be explored. Which include recombinant human penetrantin-2 (PTX-2), anti-connective tissue growth factor (CTGF) antibodies, lysophosphatidic acid (LPA) inhibitors, and phosphodiesterase 4 (PDE4) inhibitors. These drugs directly or indirectly participate in regulating the release of pulmonary inflammatory factors, epithelial cell damage and activation, and fibroblast aggregation and differentiation, thereby maintaining lung function in IPF patients. Pirfenidone and nintedanib are the only two FDA approved drugs clinically available. While these two drugs can effectively delay the decline in lung function and the progression of fibrosis, they cannot cure or reverse idiopathic pulmonary fibrosis, therefore their efficacy is poor for patients with severe IPF. Furthermore, the high market price of these drugs and the need for continuous medication place a heavy economic burden on IPF patients and their families. Therefore, there is an urgent need in clinical practice for new therapies.

Therefore, there is a need in this field for a new drug to treat idiopathic pulmonary fibrosis.

### SUMMARY OF THE INVENTION

The purpose of the present invention is to provide use of annexin A5 in the treatment of pulmonary fibrosis, in particular, idiopathic pulmonary fibrosis.

In the first aspect of the present invention, provided is a use of annexin A5 in the preparation of a composition for the prevention and/or treatment of idiopathic pulmonary fibrosis.

In another preferred embodiment, the idiopathic pulmonary fibrosis is an idiopathic pulmonary fibrosis induced by one or more factors selected from the group consisting of: infection, environmental exposure, smoking aging, genetics, and gene mutations.

In another preferred embodiment, the idiopathic pulmonary fibrosis is idiopathic pulmonary interstitial fibrosis.

In another preferred embodiment, the idiopathic pulmonary fibrosis is an idiopathic pulmonary fibrosis induced by micro-damage to alveolar epithelial cells.

In another preferred embodiment, the idiopathic pulmonary fibrosis is an idiopathic pulmonary fibrosis induced by drug treatment.

In another preferred embodiment, in the idiopathic pulmonary fibrosis induced by drug treatment, the drug is selected from the group consisting of: targeted drugs, immunotherapeutic drugs, chemical drugs, traditional Chinese medicine, or a combination thereof.

In another preferred embodiment, the drug is an anticancer drug or an antibiotic, such as bleomycin.

In another preferred embodiment, the fibrosis occurs in one or more of the following sites: bronchi, bronchioles, terminal bronchioles, respiratory bronchioles, pulmonary interstitium, and pulmonary arterioles, or perivascular regions thereof.

In another preferred embodiment, the prevention and/or treatment of idiopathic pulmonary fibrosis comprises one or more features selected from the group consisting of:
(1) improvement of lung function or maintenance of normal lung physiological function, preferably improvement of the reduction of forced vital capacity;
(2) potential of maintaining normal lung injury repair;
(3) reduction of inflammatory cell infiltration, wherein the inflammatory cell infiltration occurs in one or more of the following sites: bronchi, bronchioles, terminal bronchioles, respiratory bronchus, alveolar ducts, alveolar sacs, alveoli, and pulmonary interstitium, or perivascular regions thereof;
(4) reduction of fibrosis, wherein the fibrosis occurs in one or more of the following sites: bronchi, bronchioles, terminal bronchioles, respiratory bronchioles, and pulmonary interstitium, or perivascular regions thereof;
(5) reduction of damage to bronchioles and pulmonary arterioles within and at the margins of lesions;
(6) inhibition of the expression of profibrotic biomarkers;
(7) inhibition of the release and/or expression of pro-inflammatory factors, wherein the release of the pro-inflammatory factors occurs in one or more of the following sites: alveolar bronchial lavage fluid or lung tissue homogenate;
(8) inhibition of the differentiation of fibroblasts or the transformation and differentiation of fibroblasts-myofibroblasts in lung tissue;
(9) inhibition of the deposition or excessive deposition of collagen in lung tissue;
(10) maintenance of the morphology of alveolar epithelial cells in lung tissue;
(11) maintenance of the expression of alveolar epithelial phenotypic markers in lung tissue;
(12) reduction of the expression of hydroxyproline in lung tissue during pulmonary fibrosis;
(13) inhibition of the expression of profibrotic factors in lung tissue.

In another preferred embodiment, the damage to bronchiolar and pulmonary arteriole comprises one or more features selected from the group consisting of:
(1) reduction of alveolar hemorrhage;
(2) reduction of the lung epithelial cell proliferation, wherein the epithelial cell proliferation occurs in one or more of the following sites: bronchioles and terminal bronchioles;
(3) reduction of the proliferation of granulation tissue on the adventitia of the tube wall, wherein the granulation tissue on the adventitia of the tube wall occurs in one or more of the following sites: bronchioles and terminal bronchioles;
(4) reduction of inflammatory cell infiltration, wherein the inflammatory cell infiltration occurs in one or more of the following sites: bronchus, bronchioles, terminal bronchioles, respiratory bronchioles, alveolar ducts, alveolar sacs, alveoli, pulmonary interstitium, and pulmonary arterioles, or perivascular regions thereof;
(5) reduction of edema, wherein the edema occurs in one or more of the following sites: bronchi, bronchioles, terminal bronchioles, and respiratory bronchioles, or perivascular regions thereof;
(6) reduction of endothelial cell shedding, wherein the endothelial cell shedding occurs in pulmonary arterioles.

In another preferred embodiment, the expression includes expression of protein and/or mRNA level.

In another preferred embodiment, the fibrosis biomarker comprises one or more of fibronectin 1 (FN1), α-smooth muscle actin (α-SMA, ACTA2), type I collagen (COL-1, COL1A1), type III collagen (COL-3, COL3A1), and type V collagen (COL-5, COL5A1).

In another preferred embodiment, the pro-inflammatory factor comprises one or more of interleukin-1β (IL-1β) and tumor necrosis factor-α (TNF-α).

In another preferred embodiment, the collagen comprises one or more of type I collagen (COL-1, COL1A1), type III collagen (COL3, COL3A1), and type V collagen (COL5, COL5A1).

In another preferred embodiment, the alveolar epithelial phenotypic marker comprises pulmonary surfactant protein C (SFTPC).

In another preferred embodiment, the maintenance of normal lung physiological function refers to a decrease of no more than 30%, preferably no more than 20%, more preferably no more than 10%, for example 8%, 5%, 2%, or 1%, compared to the physiological function of a normal lung.

In another preferred embodiment, the maintenance of the morphology of alveolar epithelial cells in the lung tissue refers to a change of no more than 30%, preferably no more than 20%, more preferably no more than 10%, for example 8%, 5%, 2%, or 1%, compared to the morphology of alveolar epithelial cells of a normal lung tissue.

In another preferred embodiment, the inhibition of the expression of pro-inflammatory factors includes the expression of TNFα and IL-1β at the mRNA level.

In another preferred embodiment, the inhibition of collagen deposition or excessive deposition in lung tissue includes inhibition of the expression of type I collagen (COL1), type II collagen (COL3) at the mRNA level.

In another preferred embodiment, the inhibition of the differentiation of fibroblasts includes the inhibition of the expression of the fibroblast differentiation marker α-SMA.

In another preferred embodiment, the alveolar epithelial hyperplasia in the idiopathic pulmonary fibrosis includes alveolar wall thickening and/or formation of hyaline membranes.

In another preferred embodiment, the prevention and/or treatment of idiopathic pulmonary fibrosis comprises one or more features selected from the group consisting of:
(1) inhibition of the expression of epithelial-mesenchymal transition (EMT) markers in alveolar epithelium;
(2) inhibition of apoptosis of alveolar epithelial cells;
(3) promotion of the proliferation of alveolar epithelial cells;
(4) promotion of the migration of alveolar epithelial cells;
(5) inhibition the expression of profibrotic factors in alveolar epithelium.

In another preferred embodiment, the alveolar epithelium is type II alveolar epithelium.

In another preferred embodiment, the alveolar epithelial cell is type II alveolar epithelial cell (ATII).

In another preferred embodiment, the inhibition of the expression of epithelial-mesenchymal transition (EMT) markers in alveolar epithelium includes inhibition of the expression of Vimentin and/or promotion of the expression of E-cadherin.

In another preferred embodiment, the profibrotic factors in the alveolar epithelium includes one or more of TNF-α, SP-A, SP-D, TGFβ1, Osteopontin, CXCL12, CCL2, MMP-1, and MMP-7.

In another preferred embodiment, the prevention and/or treatment of idiopathic pulmonary fibrosis includes reduction of the expression of pro-inflammatory factors IL-6 and IL-17A in lung tissue and/or increase of the expression of anti-inflammatory factors (such as IL-10).

In another preferred embodiment, the prevention and/or treatment of idiopathic pulmonary fibrosis comprises one or more features selected from the group consisting of:
(1) the potential of maintenance normal lung injury repair;
(2) protection of normal lung interstitial structure;
(3) retardation of the decrease in forced vital capacity (FVC);
(4) retardation of interstitial lung disease;
(5) inhibition of excessive deposition of collagen in lung tissue;
(6) inhibition of fibroblasts-myofibroblasts transition in lung tissue;
(7) inhibition of the epithelial-mesenchymal transition in lung tissue;
(8) inhibition of the apoptosis of alveolar epithelium in lung tissue;
(9) promotion of the migration of alveolar epithelium in lung tissue;
(10) promotion of the proliferation of alveolar epithelium in lung tissue.

In another preferred embodiment, the composition comprises a pharmaceutical composition.

In another preferred embodiment, the composition further comprises a pharmaceutically acceptable carrier.

In another preferred embodiment, the carrier is selected from the group consisting of: solubilizers, cosolvents, antioxidants, anti-photolysis agents, pH adjusters, emulsifiers, antibacterial preservatives, complexing agents, fillers, adhesives, disintegrants, and lubricants.

In another preferred embodiment, the composition further comprises an additional drug for preventing and/or treating idiopathic pulmonary fibrosis.

In another preferred embodiment, the additional drug for preventing and/or treating idiopathic pulmonary fibrosis is selected from the group consisting of pirfenidone, nintedanib, or a combination thereof.

In another preferred embodiment, the dosage form of the composition includes a solid dosage form, a liquid dosage form, or a semi-solid dosage form.

In another preferred embodiment, the dosage form of the composition includes tablet, lozenge, powder, granule, capsule, injection, tincture, oral liquid, powder inhaler, aerosol, or spray.

In another preferred embodiment, the dosage form of the composition is a preparation for respiratory administration, such as an airway nebulized agent.

In another preferred embodiment, the dosage form of the composition is an injection, such as intravenous injection, intramuscular injection, subcutaneous injection, or intraperitoneal injection.

In another preferred embodiment, the composition is administered by injection, nebulized inhalation, or oral administration.

In another preferred embodiment, in the composition, mass percent of the annexin A5 is 0.1-99.9 wt%, preferably 1-99.9 wt%, for example 10 wt%, 20 wt%, 30 wt%, 40 wt%, 50 wt%, 60 wt%, 70 wt%, 80 wt%, or 90 wt%.

In the second aspect of the present invention, provided is a method for preventing and/or treating idiopathic pulmonary fibrosis, wherein the method comprising: administering annexin A5 to a subject in need thereof.

In another preferred embodiment, the subject comprises human or non-human mammal.

In another preferred embodiment, the non-human mammal comprises pig, cattle, sheep, rat, mouse, or rabbit.

It should be understood that, within the scope of the present invention, each technical feature of the present invention described above and each technical feature specifically described below (such as in the examples) can be combined with each other to form new or preferred technical solutions. Due to space limitations, they will not be described in detail here.

### BRIEF DESCRIPTION OF THE DRAWINGS

Figure 1 shows the results of the expression of lung fibrosis-promoting factors at the mRNA level in alveolar lavage fluid (BALF) of each group of animals in Example 1. ^{&}*p* < 0.05 vs. blank control group; ^{*}*p* < 0.05 vs. model control group.
Figure 2 shows the results of white blood cell counts in the alveolar lavage fluid (BALF) of each group of animals in Example 1. ^{&}*p* < 0.05 vs. blank control group; ^{*}*p* < 0.05 vs. model control group.
Figure 3 shows the results of the detection of hydroxyproline content in each group in Example 1. ^{&}*p* < 0.05 vs. blank control group; ^{*}*p* < 0.05, ^{**}*p* < 0.01 vs. model control group.
Figure 4 shows the results of collagen deposition and fibrosis marker expression at mRNA level in lung tissue homogenates of each group in Example 1. ^{&}*p* < 0.05 vs. blank control group; ^{*}*p* < 0.05 vs. model control group.
Figure 5 shows the results of H&E pathological staining and related scoring of the left lung in each group in Example 1. Note: a: arterioles; b: bronchioles; black arrow: inflammatory cell infiltration. ^{&&&&}*p* < 0.0001 vs. blank control group; ^{*}*p* < 0.05, ^{**}*p* < 0.01 vs. model control group.
Figure 6 shows the results of H&E pathological staining and related scoring of the left lung in each group in Example 1. Note: a: arterioles; b: bronchioles; black arrows: inflammatory cell infiltration. ^{&&&&}*p* < 0.0001 vs. blank control group; ^{*}*p* < 0.05, ^{**}*p* < 0.01 vs. model control group.
Figure 7 shows the results of Masson staining and related scoring for each group in Example 1. Note: green arrow: normal alveolar wall; blue arrow: fibrous tissue deposition; yellow arrow: partial disappearance of alveolar structure, thickening of the remaining alveolar wall; brown structure: intact alveolar wall structure, thickening of the alveolar wall. ^{&&&&}*p* < 0.0001 vs. blank control group; ^{*}*p* < 0.05, ^{**}*p* < 0.01 vs. model control group.
Figure 8 shows the results of the effects of each group in Example 2 on the cell morphology of human type II lung epithelial cells induced by silica. ^{&&}*p* < 0.01 vs. blank control group; ^{*}*p* < 0.05, ^{**}*p* < 0.01 vs. model control group.

### DETAILED DESCRIPTION

Through extensive and intensive research, the inventors unexpectedly discovered that annexin A5 has a therapeutic effect on idiopathic pulmonary fibrosis. Through systematic animal and cell experiments, the present invention demonstrates that the annexin A5 disclosed in the present invention, administered via nebulizer, has excellent therapeutic and/or delaying effects on bleomycin-induced rat idiopathic pulmonary fibrosis model. Simultaneously, cell experiments show that A5 has a protective effect against type II epithelial damage at the initial stage of silica-induced pulmonary fibrosis, effectively maintaining the morphology of type II epithelial cells. Therefore, annexin A5 is expected to become an effective means for treating idiopathic pulmonary fibrosis. On this basis, the inventors completed the present invention.

### Terms

Unless otherwise defined, all technical and scientific terms used herein have the same meaning as commonly understood by one of ordinary skill in the art to which the present invention belongs.

As used herein, the terms "comprise" "include" and "contain" are used interchangeably and include not only closed definition but also semi-closed and open definitions. In other words, the terms include "consisting of" and "substantially consisting of".

For the "promotion" or "enhancement" of a certain indicator as described in the present invention, it means that, compared with the absence of annexin A5, annexin A5 increases the indicator by, for example, at least about 10%, at least about 30%, at least about 50%, or at least about 80%.

For the "inhibition" or "reduction" of a certain indicator as described in the present invention, it means that, compared with the absence of annexin A5, annexin A5 reduces the indicator by, for example, at least about 10%, at least about 30%, at least about 50%, or at least about 80%.

As used herein, the term "treatment of idiopathic pulmonary fibrosis" does not require a 100% cure and can include alleviating disease progression and reducing the severity of idiopathic pulmonary fibrosis.

### Annexin A5

Annexin family (abbreviated as Anx) is the sensor of calcium ions in eukaryotic cell, can reversibly binds to membrane phospholipids under calcium-activated conditions. The annexin family is diverse, with 12 distinct annexin genes (ANXA1-ANXA11 and ANXA13) scattered throughout the human genome (chromosomes 1, 2, 4, 5, 8-10, and 15). The annexin family possesses a unique -COOH core structure composed of four highly homologous annexin repeat sequences. Each repeat sequence contains multiple 5α-helical structures, which, when linked with short loops, form a slightly curved plane. Annexins bind to Ca²+ on the convex plane and undergoes a conformational change that allows the hydrophilic sites at the center of the repeat sequence to bind to negatively charged phospholipids on the surrounding cell membrane, participating in cell membrane damage and repair. Studies have shown that annexins play an important role in various steps of the membrane repair system, mediated by mechanisms such as exocytosis (e.g., ANXA2), endocytosis (e.g., ANXA1, 2, 6, 8), and microparticle shedding (e.g., ANXA1, 6, 7).

AnxA5 is currently mainly used as a reagent for detecting apoptosis. In vitro studies have found that although it has multiple functions such as anti-inflammation, promoting fibrinolysis, and antithrombosis, its role in the pathogenesis of idiopathic pulmonary fibrosis (IPF) is not well understood. Annexin A5, due to its potential to maintain normal lung damage repair, provides a direction for alleviating the progression of fibrosis and is expected to become a new agent for the treatment of IPF.

The annexin A5 used in the present invention is not particularly limited and can be an annexin A5 derived from any organism, preferably from mammals (such as primates), and more preferably from human, monkey, rat, or mouse. The annexin of the present invention can also be a functional analogue, such as a protein having 50%, 60%, 70%, 75% or more, such as 80%, 85% or more, 90% or more, or even more preferably 95%, or 99% or more of identity to human annexin.

Furthermore, it should be understood that "annexin A5" includes wild-type or mutant (including truncated) annexin A5, as long as the mutant annexin A5 retains or maintains the detoxification activity of wild-type annexin A5, it can also be used in the present invention. In addition, annexin can also be a polymer of natural annexin, a fusion protein, or a chemically modified variant (such as PEG-modified) thereof, provided that these variants possess the activity of wild-type annexin. The annexin A5 in the present invention was constructed and extracted by the applicant according to the following sequence: the human annexin A5 sequence is Accession: P08758, Version: P08758.2.

### Idiopathic pulmonary fibrosis

Research generally suggests that multiple factors, including infection, environmental exposure, smoking, genetic mutations, and aging, can trigger micro-damage to alveolar epithelial cells, inducing fibrosis. Abnormally activated alveolar epithelial cells secrete large amounts of profibrotic factors. These factors, on the one hand, induce further damage to epithelial cells. On the other hand, through paracrine pathways, these factors promote fibroblasts to aggregate to the site of damage and to proliferate and differentiate into highly contractile myofibroblasts, and ultimately promote the deposition of extracellular matrix (ECM).

After injury, abnormally activated epithelial cells secrete some profibrotic regulatory factors, driving the formation of highly contractile myofibroblasts. In this case, abnormal alveolar epithelium contributes to extracellular matrix deposition and disease progression. These profibrotic mediators mainly include growth factors, matrix metalloproteinases (MMPs), chemokines, and coagulation factors. Inflammatory factors are highly expressed after lung epithelial cell damage and are widely detected in idiopathic pulmonary fibrosis.

However, unlike pneumonia, high expression of inflammation is a necessary but insufficient condition in idiopathic pulmonary fibrosis, and **anti-inflammation** alone is not sufficient to intervene in the progression of idiopathic pulmonary fibrosis.

Although the pathogenesis of idiopathic pulmonary fibrosis (IPF) remains controversial, it is generally believed that the occurrence and development of IPF disease are induced by epithelial-driven micro-damage. Micro-damage to alveolar epithelial cells, particularly type II epithelial cells, ultimately leads to activation of myofibroblasts. Abnormally activated myofibroblasts promote the deposition of extracellular matrix, which is currently the only diagnostic criterion for idiopathic pulmonary fibrosis.

### Use

The present invention unexpectedly discovered that annexin A5 can effectively treat/alleviate idiopathic pulmonary fibrosis, reduce damage to bronchioles and pulmonary arterioles within the lesion and at the lesion margin, suppress inflammatory responses, remodel lung function, reduce the severity of interstitial lung disease, inhibit fibroblast differentiation and collagen deposition, inhibit epithelial-mesenchymal transition (EMT), maintain the normal morphology of alveolar epithelial, reduce the differentiation of pulmonary fibroblast, and prevent excessive deposition of collagen, thus annexin A5 has the effect for preventing and/or treating idiopathic pulmonary fibrosis.

Specifically, the present invention provides the use of annexin A5 in the preparation of a pharmaceutical composition for preventing and/or treating idiopathic pulmonary fibrosis.

The pharmaceutical composition is a pharmaceutical composition for treating common types of idiopathic pulmonary fibrosis, specifically including idiopathic pulmonary fibrosis induced by a variety of factors such as infection, environmental exposure, smoking, aging, genetics, and gene mutation.

The alleviation of the progression of idiopathic pulmonary fibrosis, and the treatment of idiopathic pulmonary fibrosis, comprises one or more features selected from the group consisting of:
(a) improvement of bleomycin-induced idiopathic pulmonary fibrosis: improvement of lung physiological function, reduction of the expression of profibrotic factors in lung tissue homogenate, reduction the degree of fibrosis, and inhibition of the differentiation of fibroblasts and the deposition of collagen in lung tissue;
(b) reduction of the expression of epithelial-mesenchymal transition (EMT) markers; inhibition of the secretion of profibrotic factors in type II alveolar epithelial cells (ATII); reduction of the apoptosis of ATII cells; enhancement of the migration of ATII cells; and promotion of the proliferation of ATII cells;
(c) for TGFβ-induced idiopathic pulmonary fibrosis: inhibition of the differentiation of human embryonic lung fibroblasts (MRC5) cells; inhibition of collagen expression.

Preferably, the treatment of pulmonary fibrosis comprises one or more features selected from the group consisting of:
(a) improvement of bleomycin-induced pulmonary fibrosis: improvement of lung physiological function, reduction of the expression of pro-inflammatory factors in lung tissue homogenate and alveolar bronchial lavage fluid, reduction of the degree of fibrotic lesion, reduction of the content of hydroxyproline in lung tissue homogenate, and inhibition of the differentiation of fibroblasts and the deposition of collagen in lung tissue;
(b) reduction of epithelial cell damage and maintenance the normal morphology of type II cells.

The pharmaceutical composition is a pharmaceutical composition for inhibiting fibrosis.

The pharmaceutical composition is a pharmaceutical composition for improving lung function in idiopathic pulmonary fibrosis.

Preferably, the pharmaceutical composition is a pharmaceutical composition that alleviates the reduction in forced vital capacity during fibrosis.

The pharmaceutical composition is a pharmaceutical composition for alleviating the severity of idiopathic pulmonary fibrosis.

Preferably, the pharmaceutical composition is a pharmaceutical composition that reduces alveolar epithelial proliferation (thickening of alveolar walls and formation of hyaline membranes), inflammatory cell infiltration (bronchials /bronchioles/terminal bronchioles/respiratory bronchioles/alveolar ducts/alveolar sacs/perivascular area), alveolar hemorrhage, etc. in idiopathic pulmonary fibrosis.

The pharmaceutical composition is a pharmaceutical composition that reduces the expression of profibrotic factors.

Preferably, the pharmaceutical composition is a pharmaceutical composition that inhibits the expression of **inflammation-related** factors such as IL-6, IL-10, and/or IL-17A in idiopathic pulmonary fibrosis.

Preferably, the pharmaceutical composition is a pharmaceutical composition that inhibits the expression of IL-1β and TNF-α in pulmonary fibrosis.

The pharmaceutical composition is a pharmaceutical composition that inhibits the differentiation of fibroblasts in lung tissue.

Preferably, the pharmaceutical composition is a pharmaceutical composition that inhibits the expression of alpha-SMA in lung tissue with idiopathic pulmonary fibrosis.

The pharmaceutical composition is a pharmaceutical composition that inhibits the content of hydroxyproline in lung tissue.

The pharmaceutical composition is a pharmaceutical composition that inhibits the deposition of collagen in lung tissue. Preferably, the pharmaceutical composition inhibits the expression of type I and type III collagen in pulmonary fibrosis.

The pharmaceutical composition is a pharmaceutical composition that protects the homeostasis of human type II alveolar epithelium.

The pharmaceutical composition is a pharmaceutical composition that inhibits the deposition of collagen in lung tissue. Preferably, the pharmaceutical composition inhibits staining of collagen in idiopathic pulmonary fibrosis.

The pharmaceutical composition is a pharmaceutical composition that protects the homeostasis of human type II alveolar epithelium.

The pharmaceutical composition is a pharmaceutical composition that inhibits human type II alveolar epithelial-mesenchymal transition (EMT).

Preferably, the pharmaceutical composition reduces the high expression of Vimentin and/or promotes the expression of E-cadherin in human type II alveolar epithelial cells during idiopathic pulmonary fibrosis.

The pharmaceutical composition is a pharmaceutical composition that inhibits the secretion of profibrotic factors in human type II alveolar epithelium.

Preferably, the pharmaceutical composition reduces the secretion of one or more profibrotic factors, TNF-α, SP-A, SP-D, TGFβ1, Osteopontin, CXCL12, CCL2, MMP-1, and MMP-7, in human type II alveolar epithelial cells during idiopathic pulmonary fibrosis.

The pharmaceutical composition is a pharmaceutical composition that reduces the apoptosis of human type II alveolar epithelial cells.

The pharmaceutical composition is a pharmaceutical composition that promotes the migration of human type II alveolar epithelial cells.

The pharmaceutical composition is a pharmaceutical composition that enhances the proliferation of human type II alveolar epithelial cells.

The pharmaceutical composition is a pharmaceutical composition that inhibits the differentiation and/or activation of human embryonic lung fibroblasts. Preferably, the pharmaceutical composition is a pharmaceutical composition that inhibits the expression of alpha-SMA in human embryonic lung fibroblasts.

The pharmaceutical composition is a pharmaceutical composition that inhibits collagen deposition and expression in human embryonic lung fibroblasts. Preferably, the pharmaceutical composition is a pharmaceutical composition that inhibits the expression of one or more of Type I collagen (COL-1), Type III collagen (COL-3), and Type V collagen (COL-5) in human embryonic lung fibroblasts.

Preferably, the pharmaceutical composition is a composition having one or more of the following functions:
(1) having the potential of maintaining normal lung injury repair;
(2) protecting normal lung interstitial structure;
(3) reducing the rate of decrease in forced vital capacity (FVC);
(4) alleviating the severity of interstitial lung disease;
(5) inhibiting excessive deposition of collagen in lung tissue;
(6) reducing the **transition** of fibroblasts-myofibroblasts in lung tissue;
(7) inhibiting the epithelial-mesenchymal transition in lung tissue;
(8) inhibiting the apoptosis level of alveolar epithelium in lung tissue;
(9) increasing the migration and proliferation level of alveolar epithelium in lung tissue.

Preferably, the pharmaceutical composition is a composition having one or more of the following functions:
(1) improving lung function or maintaining normal physiological function of lung;
(2) reducing fibrosis, wherein the fibrosis occurs in one or more of the following sites: the bronchi, bronchioles, terminal bronchioles, respiratory bronchioles, pulmonary interstitium, and pulmonary arterioles or perivascular regions thereof;
(3) reducing bronchioles and pulmonary arterioles damage within the lesion and at the lesion margins;
(4) inhibiting the expression of profibrotic biomarkers;
(5) inhibiting the release of pro-inflammatory factors, wherein the release of the pro-inflammatory factors occurs in one or more of the following sites: alveolar bronchial lavage fluid and lung tissue homogenate;
(6) inhibiting the differentiation of fibroblast;
(7) inhibiting the deposition of collagen in lung tissue;
(8) maintaining the morphology of alveolar epithelial cells in the lung tissue;
(9) maintaining the expression of alveolar epithelial phenotypic markers in lung tissue;
(10) reducing the expression of hydroxyproline in alveolar epithelial hyperplasia tissue in pulmonary fibrosis.

### Composition and administration

The compositions of the present invention include (but are not limited to): pharmaceutical compositions, health care compositions, dietary supplements, etc.

Typically, the cell-free fat extract of the present invention can be prepared into pharmaceutical compositions, such as tablets, capsules, powders, microparticles, solutions, lozenges, injections, spirits, suspensions, tinctures, poultices, liniments, lotions, and aerosols. The pharmaceutical compositions can be prepared using commonly known preparation techniques, and suitable pharmaceutical additives can be added into the pharmaceutical composition.

The compositions of the present invention may also include pharmaceutically, nutraceutically, or dietetically acceptable carriers. "Pharmaceutically, nutraceutically, or dietetically acceptable carriers" refers to one or more compatible solid or liquid fillers or gelling substances suitable for human use and possessing sufficient purity and sufficiently low toxicity. "Compatibility" used herein refers to each component in the composition can mixed with the compounds of the present invention and with each other without significantly reducing the efficacy of the compounds. Examples of pharmaceutically, nutraceutically, or dietetically acceptable carriers include cellulose and derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerin, mannitol, sorbitol, etc.), emulsifiers (such as Tween^{®}), wetting agents (such as sodium dodecyl sulfate), colorants, flavoring agents, stabilizers, antioxidants, preservatives, pyrogen-free water, etc.

There are no particular limitations on the administration means of the composition of the present invention. Representative administration means include (but are not limited to): oral administration, parenteral administration (intravenous administration, intramuscular administration), topical administration, and inhalation administration. Preferred administration means are injection administration and inhalation administration.

The dosage forms of the compositions or preparations of the present invention are oral preparations, topical , or injectable preparations. Representatively, solid dosage forms for oral administration or application include capsules, tablets, pills, powders, and granules. In these solid preparations, the active compound is mixed with at least one conventional inert excipient (or carrier), such as sodium citrate or dicalcium phosphate, or mixed with the following components: (a) fillers or compatibilizers, such as starch, lactose, sucrose, glucose, mannitol, and silica; (b) binders, such as hydroxymethyl cellulose, alginate, gelatin, polyvinylpyrrolidone, sucrose, and gum arabic; (c) humectants, such as glycerin; (d) disintegrants, such as agar, calcium carbonate, potato starch or cassava starch, alginic acid, certain complex silicates, and sodium carbonate; (e) slow-release agents, such as paraffin; (f) absorption accelerators, such as quaternary ammonium compounds; (g) wetting agents, such as cetyl alcohol and glyceryl monostearate; (h) adsorbents, such as kaolin; and (i) lubricants, such as talc, calcium stearate, magnesium stearate, solid polyethylene glycol, sodium dodecyl sulfate, or mixtures thereof. Buffer agents may also be included in capsules, tablets, and pills.

Solid dosage forms such as tablets, sugar pills, capsules, pellets, and granules can be prepared using coatings and shell materials, such as casings and other materials known in the art. They may contain opacifiers.

Liquid dosage forms for oral administration or application include pharmaceutically acceptable emulsions, solutions, suspensions, syrups, or tinctures. In addition to the active compound, liquid dosage forms may contain inert diluents conventionally used in the art, such as water or other solvents, solubilizers and emulsifiers, e.g., ethanol, isopropanol, ethyl carbonate, ethyl acetate, propylene glycol, 1,3-butanediol, dimethylformamide, and oils, particularly cottonseed oil, peanut oil, corn germ oil, olive oil, castor oil, and sesame oil, or mixtures thereof.

In addition to these inert diluents, the composition may also contain auxiliaries such as wetting agents, emulsifiers and suspending agents, sweeteners, flavoring agents and fragrances.

In addition to the active ingredient, the suspension may contain suspending agents, such as ethoxylated isostearyl alcohol, polyoxyethylene sorbitol and dehydrated sorbitol esters, microcrystalline cellulose, aluminum methoxide and agar, or mixtures of these substances, etc.

Compositions for parenteral injection may comprise physiologically acceptable sterile aqueous or anhydrous solutions, dispersions, suspensions, or emulsions, and sterile powders for redissolving into sterile injectable solutions or dispersions. Suitable aqueous and non-aqueous carriers, diluents, solvents, or excipients include water, ethanol, polyols, and suitable mixtures thereof.

Dosage forms of the compositions of the present invention for topical application or administration include ointments, powders, patches, sprays, and inhalants. The active ingredient is mixed under sterile conditions with a physiologically acceptable carriers and any preservatives, buffers, or propellants that may be needed when necessary.

The compositions of the present invention can be administered alone or in combination with other drugs for the prevention and/or treatment of idiopathic pulmonary fibrosis.

When administering the composition, a safe and effective amount of the cell-free lipid extract of the present invention is applied to the human or non-human animal (such as rat, mouse, dog, cat, cow, sheep, chicken, duck, etc.) in need of treatment, wherein the dosage administered is a pharmaceutically, dietetically, or nutraceutically acceptable effective dosage. As used herein, the term "safe and effective amount" refers to an amount that produces function or activity in humans and/or animals and is acceptable to humans and/or animals. Those skilled in the art will understand that the "safe and effective amount" may vary depending on the form of the pharmaceutical composition, the route of administration, the excipients used, the severity of the disease, and the combination with other drugs. For example, for a person weighing 60 kg, the daily dosage is typically 0.1-1000 mg, preferably 1-600 mg, and more preferably 2-300 mg. Of course, the specific dosage should also consider factors such as the route of administration and the patient's health condition, which are within the scope of a skilled physician's expertise.

### The main advantages of the present invention include:

The present invention discloses for the first time the use of annexin A5 in the prevention and/or idiopathic pulmonary fibrosis. Through systematic animal and cell experiments, the present invention demonstrates that the annexin A5 disclosed herein has effects of treating/delaying idiopathic pulmonary fibrosis through inhalation via an atomizing needle.

The present invention will be further illustrated below with reference to specific examples. It should be understood that these examples are for illustrative purposes only and are not intended to limit the scope of the invention. Experimental methods in the following embodiments, unless otherwise specified, are generally performed under conventional conditions or as recommended by the manufacturer. Unless otherwise stated, percentages and parts are weight percentages and parts by weight.

The annexin that can be used in the present invention has no particular limitation and can be derived from any organism, preferably from mammals (such as primates), and more preferably from humans, monkeys, rats, or mice. The annexin of the present invention can also be a functional analogue, such as a protein having 50%, 60%, 70%, 75% or more, such as 80%, 85% or more, 90% or more, or even more preferably 95% or 99% or more identity to human annexin. It should be understood that "annexin" includes wild-type or mutant (including truncated) annexin, as long as the mutant annexin retains or maintains the detoxification activity of wild-type annexin, it can also be used in the present invention. Furthermore, the annexin can also be a polymer of natural annexin, a fusion protein, or a chemically modified variant (such as PEG-modified), provided that these variants possess the activity of wild-type annexin. Annexin A5 in the present application was extracted and constructed by a company according to the following sequence.

The sequence of human annexin A5 is as follows: sequence of Annexin with Accession number P08758

MAQVLRGTVTDFPGFDERADAETLRKAMKGLGTDEESILTLLTSRSNAQRQEIS
AAFKTLFGRDLLDDLKSELTGKFEKLIVALMKPSRLYDAYELKHALKGAGTNEKVL
TEIIASRTPEELRAIKQVYEEEYGSSLEDDVVGDTSGYYQRMLVVLLQANRDPDAGI
DEAQVEQDAQALFQAGELKWGTDEEKFITIFGTRSVSHLRKVFDKYMTISGFQIEETI
DRETSGNLEQLLLAVVKSIRSIPAYLAETLYYAMKGAGTDDHTLIRVMVSRSEIDLF
NIRKEFRKNFATSLYSMIKGDTSGDYKKALLLLCGEDD (SEQ ID No: 1)

### Example 1: Effect of Annexin A5 on bleomycin-induced pulmonary fibrosis in rats

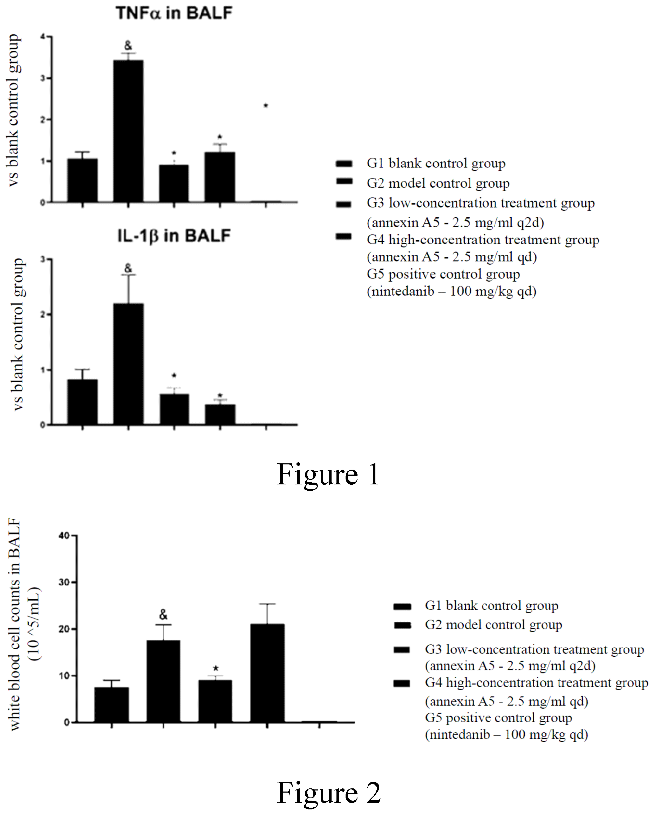

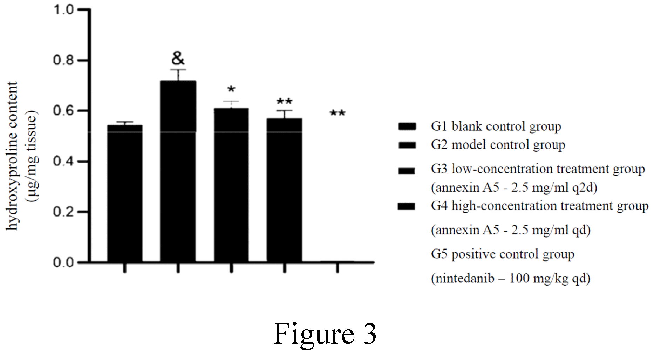

1. Experimental animals: thirty-five healthy male SD rats were housed in an SPF-grade barrier system, with body weight of 270-290 g at the time of experiment. Animal Use Certificate Number: SYXK (Zisu) 2022-0005. The temperature, humidity, and light control system followed international standards. The experimental animal protocol was jointly approved and confirmed by the IACUC committee. Management and operation were strictly carried out in accordance with relevant standard operating procedures (SOPs).
2. Experimental materials:
   The experimental equipment included: electronic balance (NVT1601B/3, OHAUS), multi-channel anesthesia machine (AMS, Gene & I), electronic balance (AUW120D, SHIMADZU), small animal ventilator (R407, RWD Life Science), surgical microscope (XT-X-4A, Xincheng Company), fully automated blood analyzer (XS800i, SYSMEX), pure water system (arium pro, Sartorius), low-temperature centrifuge (Legend Micro17R, Thermo Scientific), numerical control ultrasonic cleaner (KQ-100DE, Kunshan Ultrasonic Instrument Co., Ltd.), drying oven (DHG-9055A, Shanghai Yiheng Scientific Instrument Co., Ltd.), microscope (ECLIPSE E100, Nikon), slice scanner (NANO Zoomer S210, HAMAMATSU), automatic staining machine (ST5020, LEICA), and paraffin embedding machine (RM2235), LEICA), paraffin microtome (RM2235, LEICA), tissue dehydrator (HistoCore PEARL, LEICA).
   The experimental reagents included: physiological saline (Zhejiang Dubang Pharmaceutical Co., Ltd.), bleomycin hydrochloride for injection (Nippon Kayaku Co., Ltd.), sodium methylcellulose (Aladdin), Tween 80 (Sigma), PBS buffer (Biosharp), meloxicam (Qilu Animal Health), hemolytic agent for blood cell analysis (Zhejiang Xinke Medical Technology Co., Ltd.), diluent for blood cell analysis (Zhejiang Xinke Medical Technology Co., Ltd.), staining solution for blood cell analysis (Zhejiang Xinke Medical Technology Co., Ltd.), BIBF1120 (Chembest), annexin A5 (Shanghai Seme Cell Technology Co., Ltd.), Tissue RNA Purification Kit PLUS (Ezbioscience), Color Reverse Transcription Kit (EZBioscience), 2× Color SYBR Green qPCR Master Mix (EZBioscience), rat fluorescent quantitative PCR primers (COL1A1/COL3A1/ACTA2/ACTB/TNFα/IL-1β) (Sangon Biotech (Shanghai) Co., Ltd.), and hydroxyproline Hyp assay kit (alkaline hydrolysis method) (Nanjing Jiancheng).
3. Experimental methods:
   3.1 Experimental animal grouping: the experiment was set up with a blank control group, a model control group, a positive control group, an experimental treatment group (low concentration), and an experimental treatment group (high concentration), with 7 rats in each group.
   3.2 Establishment of a bilateral pulmonary fibrosis model in rats: the model was established twice, on Day 1 and Day 8. The day of model establishment was designated as Day 1. The animals were weighed the day before modeling and randomly divided into a G1 sham model group (n=7) and a model animal group (n=28). The model animal group was further divided into a model control group (n=7), a positive control group (n=7), an experimental treatment group (low concentration, n=7), and an experimental treatment group (high concentration, n=7). After the rats were anesthetized by inhalation with 2.5% isoflurane, a double-blind model was established to create a bilateral pulmonary fibrosis model in rats. The surgical access to the neck of all animals in the model control group, positive control group, experimental treatment group (low concentration), and experimental treatment group (high concentration) were opened and the models were established by administering with bleomycin (1.5 mg/kg, 1 mL/kg) via tracheal injection. The wounds were sutured after surgery. After the surgery, the animals were kept warm under a 37 °C electric blanket until they were fully awake. Once they were confirmed to be able to eat and drink freely, they were returned to their rearing cages for normal rearing. Meloxicam was administered subcutaneously for three consecutive days to relieve pain. On Day 8, the model animal group (n=28) were administered with bleomycin (1.5 mg/kg) via tracheal infusion again, and all the above modeling steps were repeated.
   3.3 Drug intervention: the intended clinical administration route was simulated. Based on the most recently measured animal weights, after modeling was completed on Day 14, animals were randomly grouped according to their weight, with 7 experimental animals in each group: G1 blank control group, G2 model control group, G3 low-concentration experimental treatment group, G4 high-concentration experimental treatment group, and G5 positive control group. After grouping, all animals were administered with drug according to the frequency and method specified in their respective groups starting from day 15 until day 28. During the experiment, changes in mouse weight, food and water intake were recorded daily, and activity levels, coat color changes, and respiratory status and the like of rats were observed daily. Wherein G1 blank control group: administered via nebulized inhalation of commercially available saline (250 µL/animal) once every two days (Q2D); G2 model control group: administered via nebulized inhalation of commercially available saline (250 µL/animal) once every two days (Q2D); G3 low-concentration experimental treatment group: administered via nebulized inhalation of annexin A5 (1.5 mg/kg, 250 µL/animal) once every two days (Q2D); G4 high-concentration experimental treatment group: administered via nebulized inhalation of annexin A5 (1.5 mg/kg, 250 µL/animal) once daily (QD); G5 positive control group: administered via nebulized inhalation of nintedanib BIBF1120 (100 mg/kg, 10 mL/kg) once daily (QD).
   3.4 Endpoint of the experiment:
      On day 29 of the experiment, after deep anesthesia with isoflurane, the animals' abdominal and thoracic cavities were opened to fully expose the lungs. The left bronchus was ligated, and the right lung was repeatedly lavaged four times with sodium chloride injection. The collected right lung lavage fluid was centrifuged, and the supernatant was collected and stored at -80 °C for later use. The centrifuged cell pellet was resuspended with PBS buffer and aliquoted into two portions. One portion was centrifuged again, and the supernatant was discarded. The cell pellet was used for RNA extraction, reverse transcription, and fluorescence quantitative PCR detection. The other portion was used for white blood cell counting and classification. After lavage of the right lung, the upper lobe, lower lobe, and accessory lobe tissues were dissected separately, without dehydration, and quickly frozen in liquid nitrogen at -80 °C for subsequent detection and determination of hydroxyproline content. The right middle lobe was homogenized using an ultrasonic homogenizer, and RNA was extracted, reverse transcribed, and used for fluorescence quantitative PCR detection. After the bloodstains in the left lung were washed away with normal saline, it was perfused with 10% formalin. After being preserved in fixative for 48 hours, it was then subjected to paraffin embedding, section staining, and pathological examination.
   3.5 Experimental results:
      1. Expression of lung fibrosis-promoting factors at the mRNA level in bronchoalveolar lavage fluid (BALF) of each group of animals: BALF cell pellets of rats were washed once with PBS, centrifuged, and RNA was extracted, reverse transcribed, and the expression of pro-inflammatory factors (IL-1β, TNFα) was detected by fluorescence quantitative PCR according to the EZB operating instructions.
         The results showed that the expression of pro-inflammatory factors (IL-1β and TNFα) in BALF was significantly increased after modeling (*p* < 0.05). Compared with the model control group, the expression of inflammatory factors in the BALF of the experimental group was reduced, with statistically significant differences. The improvement efficiency was comparable to that of the positive control group (nintedanib). Specific results are as follows: as shown in Figure 1, compared with the blank control group, the expression of pro-inflammatory factor (IL-1β) in the bronchoalveolar lavage fluid (BALF) of rats in the model control group was significantly increased at the mRNA level (*p* < 0.05); compared with the model control group, the expression in the BALF of the low-concentration experimental treatment group, high-concentration experimental treatment group, and positive control group was significantly reduced (*p* < 0.05). Compared with the positive control group, the experimental treatment group showed better ability to inhibit the expression of IL-1β at the mRNA level. As shown in Figure 1, compared with the blank control group, the expression of pro-inflammatory factors (TNFα) in the bronchoalveolar lavage fluid (BALF) of rats in the model control group was significantly increased at the mRNA level (*p* < 0.05); compared with the model control group, the expression of TNFα in BALF of the low-concentration experimental treatment group and high-concentration experimental treatment group was significantly decreased (*p* < 0.05). Compared with the positive control group, the experimental treatment group had a better ability to inhibit the expression of TNFα at the mRNA level.
      2. White blood cell count in bronchoalveolar lavage fluid (BALF) of animal in each group: BALF lavage fluid was collected at the end of the experiment, centrifuged and resuspended, and inflammatory cells were classified and counted.
         The results are shown in Figure 2, compared with the blank control group, the white blood cell count (WBC) in BALF was significantly increased after modeling (*p* < 0.05); compared with the model control group, the white blood cell count (WBC) in BALF of the low-concentration experimental treatment group was significantly decreased (*p* < 0.05), while the WBC count in BALF of the high-concentration experimental treatment group and the positive control group showed no significant change (*p* < 0.05). Compared with the positive control group, the low-concentration experimental treatment group had a better ability to reduce the white blood cell count. The positive control group even showed a further increase in white blood cell count compared to the model control group.
      3. Detection of hydroxyproline content: after lavage of the right lung, tissue samples from the upper lobe, lower lobe, and accessory lobe were taken and processed according to the instructions of the hydroxyproline content detection kit to determine the hydroxyproline content in the lung tissue.
         The results showed that the hydroxyproline content in all groups of animals significantly increased after modeling. Compared with the model control group, the mean hydroxyproline content of the treatment groups was reduced with statistical differences, and the improvement efficiency thereof was comparable to that of the positive control group (nintedanib). Specific results are shown in Figure 3, compared with the blank control group, the hydroxyproline content in the lung tissue of rats in the model control group was significantly increased (*p* < 0.05); compared with the model control group, the hydroxyproline contents in the lung tissue of the low-concentration experimental treatment group, the high-concentration experimental treatment group, and the positive control group were significantly decreased (*p* < 0.05, *p* < 0.01, *p* < 0.01).
      4. Collagen deposition and expression of fibrosis biomarkers at the mRNA level in lung tissue homogenate: a portion of right lung tissue was taken and treated according to the instructions of the tissue RNA extraction kit and RT-PCR detection kit to detect the expression of typical fibrosis biomarkers (ACTA2/COL1A1/COL3A1) at the mRNA level in lung tissue homogenate.

The results showed that collagen deposition and fibrosis markers at the mRNA level in lung tissue homogenates of all groups of animals were significantly increased after modeling. Compared with the model control group, the expression of collagen deposition and fibrosis markers at the mRNA level can be significantly inhibited in the experimental group animals with statistical differences. The inhibitory efficiency thereof was comparable to that of the positive control group (nintedanib).

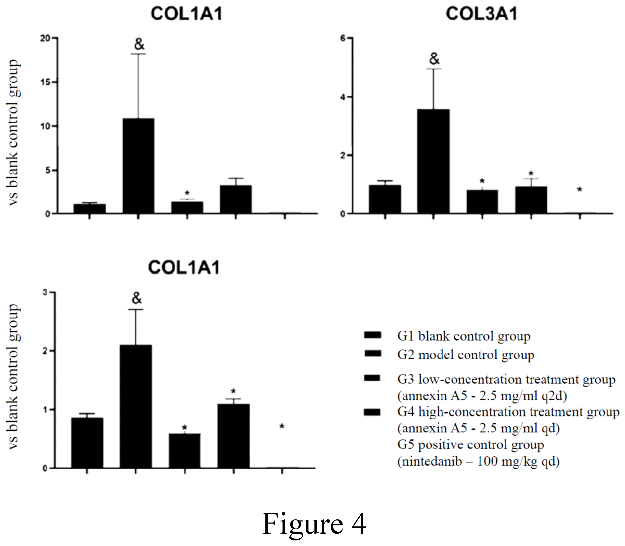

The specific results are shown in Figure 4, compared with the blank control group, the expression of type I collagen COL1A1 at the mRNA level in the lung tissue homogenate of rats in the model control group was significantly increased (*p* < 0.05); compared with the model control group, the expression in the low-concentration experimental treatment group was significantly decreased (*p* < 0.05). Compared with the positive control group, the experimental treatment group was more effective in reducing the expression of COL1A1 at the mRNA level.

The results are shown in Figure 4, compared with the blank control group, the expression of type III collagen COL3A1 at the mRNA level in the lung tissue homogenate of rats in the model control group was significantly increased (*p* < 0.05); compared with the model control group, the expression in the low-concentration experimental treatment group was significantly decreased (*p* < 0.05); compared with the model control group, the expression in the high-concentration experimental treatment group was significantly decreased (*p* < 0.05); compared with the model control group, the expression in the positive control group was significantly decreased (*p* < 0.05). Compared with the positive control group, the experimental treatment group had a comparable effect in reducing the expression of COL3A1 at the mRNA level.

The results are shown in Figure 4, compared with the blank control group, the expression of the fibrosis marker α-SMA (ACTA2) in the lung tissue homogenate of rats in the model control group was significantly increased at the mRNA level (*p* < 0.05); compared with the model control group, the expression in the low-concentration experimental treatment group was significantly decreased (p < 0.05). Compared with the model control group, the expression in the high-concentration experimental treatment group was significantly decreased (*p* < 0.05); compared with the model control group, the expression in the positive control group was significantly decreased (*p* < 0.05). Compared with the positive control group, the experimental treatment groups showed comparable effect in inhibiting the expression of the fibrosis marker α-SMA (ACTA2) at the mRNA level.

5. Left lung H&E pathological staining: paraffin blocks were prepared from the left lung tissue, sectioned (3-4 µm), and subjected to H&E staining. H&E staining: lung tissue sections were dewaxed to water (xylene, anhydrous ethanol, 90% ethanol dehydration, water washing). Then, the lung tissue sections were subjected to hematoxylin staining, hydrochloric acid differentiation, water washing, and eosin staining. Finally, dehydration and clearing were carried out (95% ethanol, anhydrous ethanol, xylene). After the treatment, neutral resin was dropped onto the tissue, and a coverslip was placed on top for sealing. The next day, the stained sections were scanned entirely using a NanoZoomer Digital Pathology (S210) slide scanner. Five fields of view were randomly and dispersedly selected within the lesion area and at the margin of the lesion area, respectively, and semi-quantitative scoring was performed seeparately for the damages and inflammatory changes of the terminal bronchioles and accompanying small pulmonary arteries. The scoring criteria are shown in Tables 1 and 2. The evaluation criteria are: alveolar epithelial proliferation (thickening of the alveolar walls and formation of hyaline membranes), inflammatory cell infiltration (bronchials/bronchioles/terminal bronchioles/respiratory bronchioles/alveolar ducts/alveolar sacs/pervascular region), alveolar hemorrhage, and congestion.

**Table 1. Pathological evaluation indicators for terminal bronchiolar damage and inflammatory infiltration**

| score | Terminal bronchiolar wall damage | Inflammatory cell infiltration of terminal bronchiolar wall |
|---|---|---|
| 0 | Normal tissue structure | Normal tissue structure with no inflammatory cell infiltration |
| 1 | Normal tissue structure with bronchial wall damage in less than 1/2 of the area, presenting as damage and regeneration of bronchial epithelium, edema of the bronchial wall, and degeneration or regeneration of the tunica media muscle layer. | Scattered inflammatory cell infiltration in the tunica externa of the tube wall, non-focal, with fewer than 10 inflammatory cells. |
| 2 | Normal tissue structure, with bronchial wall damage in more than 1/2 of the area, presenting as damage and regeneration of bronchial epithelium, edema of the bronchial wall, and degeneration or regeneration of the tunica media muscle layer. | Scattered and abundant inflammatory cell infiltration in the tunica externa of the tube wall, focal (single or multiple), totaling less than 1/2 of the wall area. |
| 3 | Normal tissue structure is normal, with bronchial wall damage in more than 1/2 of the area, presenting as damage and regeneration of bronchial epithelium, edema of the bronchial wall, degeneration or regeneration of the tunica medi muscle layer, and adventitial granuloma formation or fibrosis. | Diffuse inflammatory cell infiltration in the tunica externa of the tube wall, totaling more than 1/2 of the wall area, or inflammatory cell infiltration in the intima and media. |

**Table 2. Pathological evaluation indicators for small pulmonary artery damage and inflammatory infiltration**

| score | Pulmonary arteriole wall damage | Inflammatory cell infiltration of pulmonary arteriole |
|---|---|---|
| 0 | Normal pulmonary arteriole structure. | Normal pulmonary arteriole structure. |
| 1 | Partial endothelial cells shedding. | Scattered inflammatory cell infiltration in the tunica externa, non-focal, with fewer than 10 inflammatory cells. |
| 2 | Endothelial cell shedding, tunica media smooth muscle degeneration, proliferation or small focal necrosis. | Scattered and abundant inflammatory cell infiltration in the tunica externa, focal (single or multiple), totaling less than 1/2 of the area of the tunica externa of the tube wall. |
| 3 | Endothelial cell shedding, tunica media smooth muscle degeneration, proliferation or small focal necrosis, adventitial granuloma formation or fibrosis. | Diffuse inflammatory cell infiltration in the tunica externa of the tube wall, totaling more than 1/2 of the area of the tube wall, or inflammatory cell infiltration in the tunica media. |

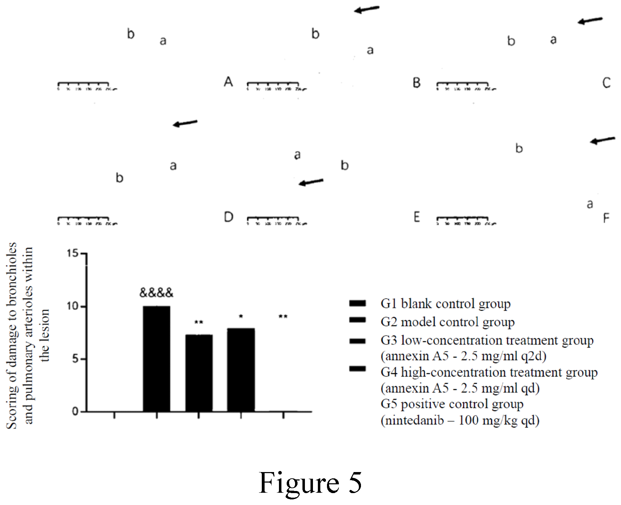

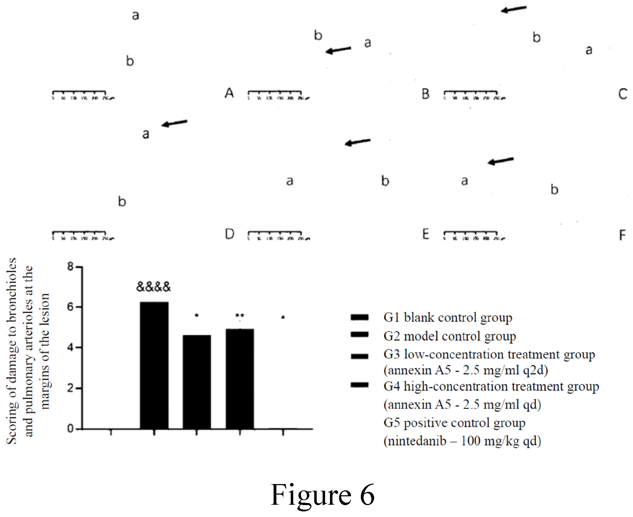

The results showed that after modeling, the experimental animals exhibited varying degrees of proliferation of epithelial cells, inflammatory cell infiltration, and proliferation of granulation tissue in the tunica externa of the tube wall in the bronchioles and terminal bronchioles within the lesion and at the lesion margins, and the pulmonary arterioles showed endothelial cell shedding and inflammatory cell infiltration (Figures 5 and 6); compared with the model control group, the treatment groups showed significant improvement in the damage of the bronchioles and pulmonary arterioles within the lesion and at the margins of lesion.

The specific results are shown in Figures 5 and 6, compared with the blank control group, the scores of bronchioles and pulmonary arterioles damage within the lesion and at the margins of lesion of the model control group were significantly increased (*p* < 0.0001, *p* < 0.0001) (Figures 5 and 6). Compared with the model control group, the scores of bronchioles and pulmonary arterioles damage within the lesion of the low-concentration treatment group, high-concentration treatment group, and positive control group were significantly decreased (*p* < 0.01, *p* < 0.05, *p* < 0.01) (Figure 5). Compared with the model control group, the scores of bronchioles and pulmonary arterioles damage at the margins of lesion of the low-concentration treatment group, high-concentration treatment group, and positive control group were significantly decreased (*p* < 0.05, *p* < 0.01, *p* < 0.05) (Figure 6). Moreover, the inhibition efficiencies of the treatment groups were comparable to that of the positive control group (nintedanib) (Figures 5 and 6).

6. Histopathological examination - Masson staining: a lung tissue section from left lung was taken and dewaxed to water (xylene, anhydrous ethanol, 90% ethanol dehydration, washing). Then, it was differentiated with Weiger's iron hematoxylin, washed with water, 0.5% hydrochloric acid ethanol, washed with water, stained with Ponceau S acid fuchsin solution, washed with water, stained with 1% phosphomolybdic acid aqueous solution, counterstained with aniline blue or brilliant green, and treated with 1% glacial acetic acid. Finally, it underwent dehydration and clearing (95% ethanol, anhydrous ethanol, xylene). After treatment, neutral resin was applied to the tissue, and a coverslip was placed to seal. The next day, the section was scanned entirely using a NanoZoomer Digital Pathology (S210) slide scanner. Ten fields of view with an area of 1 mm² were randomly selected within the lesion area, and a semi-quantitative scoring was performed by the pathologist under double-blind conditions according to the Ashcroft scoring system (Table 3).

**Table 3 Pathological evaluation indicators for pulmonary fibrosis**

| Fibrosis grading | Ashcroft scoring criteria |
|---|---|
| 0 | Alveolar septa: no fibrotic lesions |
| | Lung structure: normal |
| 1 | Alveolar septa: isolated simple fibrotic change (alveolar septal thickening, but less than three times that of the normal lung). |
| | Lung structure: partial enlargement of alveolar spaces, a small amount of exudate, no fibrotic material present. |
| 2 | Alveolar septa: definite fibrotic changes (alveolar septal thickening, greater than three times that of the normal lung), forming small nodules, but not connected. |
| | Lung structure: partial enlargement of alveolar spaces, a small amount of exudate, no fibrotic material present. |
| 3 | Alveolar septa: non-interrupted fibrosis visible on almost all alveolar walls per high-power field (alveolar septal thickening greater than three times that of the normal lung). |
| | Lung structure: partial enlargement of alveolar spaces, a small amount of exudate, no fibrotic material present. |
| 4 | Alveolar septa: alveolar septa are still observable |
| | Lung structure: isolated fibrotic nodules in the alveolar spaces (≤10% of high-power fields). |
| | Alveolar septa: alveolar septa are still observable |
| 5 | Lung structure: confluent fibrotic nodules in the alveolar spaces (>10% and ≤50% of high-power fields). Severe damage to lung tissue structure, but structure still remains. |
| 6 | Alveolar septa: observable, but almost non-existent. |
| 7 | Lung structure: large uninterrupted fibrotic nodules (>50% of high-power fields). The lung tissue framework is almost non-existent. |
| | Alveolar septa: no longer exist. |
| | Lung structure: alveolar spaces almost completely filled with fibrotic material, but still with less than five vacuole-like structures. |
| 8 | Alveolar septa: no longer exist. |
| | Lung structure: alveolar spaces completely filled with fibrotic tissue under high-power field. |

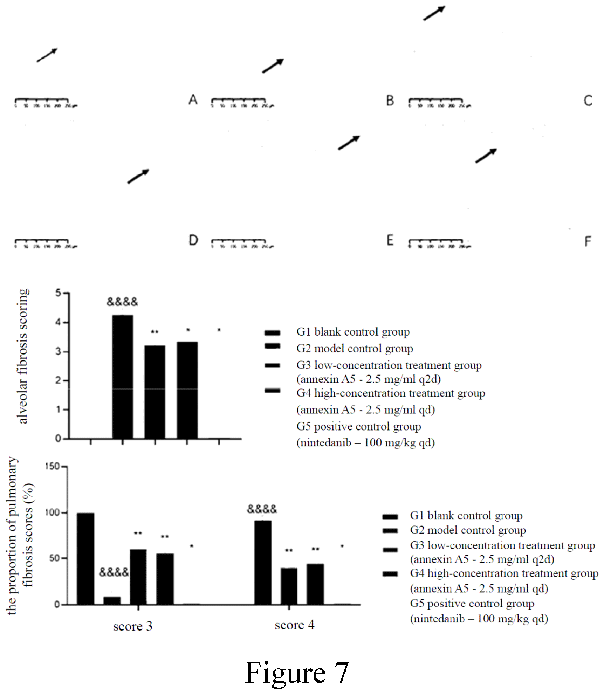

Masson staining results showed varying degrees of fibrous tissue deposition in the model group and each treatment groups, with partial loss of alveolar structure and thickening of alveolar walls (Figure 7). Ashcroft scoring showed that compared with the blank control group, the pulmonary fibrosis score of the model control group was significantly increased (*p* < 0.0001), compared with the model control group, the pulmonary fibrosis scores of the low-concentration treatment group, the high-concentration treatment group, and the positive control group were significantly decreased (*p* < 0.01, *p* < 0.05, *p* < 0.05) (Figure 7). Compared with the positive control group, the experimental treatment groups showed comparable ability to reduce pulmonary fibrosis scores (Figure 7). Using the Ashcroft score of 3 as the cutoff, the percentage of pulmonary fibrosis with a score of 3 or below (including 3) and a score of 4 or above (including 4) was calculated. The results showed that 91.43% of the lesion areas in the model control group had a score of 4 or above. Compared with the model control group, the proportions of fibrosis scores ≤3 of the low concentration treatment group, the high concentration treatment group, and the positive control group were significantly increased (*p* < 0.001, *p* < 0.01, *p* < 0.01), while the proportion of scores ≥4 were significantly decreased (*p* < 0.001, *p* < 0.01, *p* < 0.01) (Figure 7).

After damage, abnormally activated epithelial cells secrete profibrotic regulatory factors, promoting the formation of highly contractile myofibroblasts. In this case, abnormal alveolar epithelium contributes to ECM deposition and disease progression. Similar to pneumonia, high expression of inflammatory factors after lung epithelial cell damage is widely detected in pulmonary fibrosis. However, the high expression of inflammation is a necessary but not sufficient condition in pulmonary fibrosis (unlike pneumonia), and anti-inflammatory therapy alone may not be sufficient to intervene in the progression of pulmonary fibrosis. Although the pathogenesis of pulmonary fibrosis remains controversial, the promotion of extracellular matrix deposition by abnormally activated myofibroblasts is currently the only criterion for diagnostic pulmonary fibrosis. Therefore, in light of the above findings, annexin A5 may play a good anti-fibrotic role. Nebulized inhalation of annexin A5 significantly improved bleomycin-induced pulmonary fibrosis in rats, with therapeutic effects comparable to nintedanib, providing a reliable basis for further clinical application of annexin A5 in the treatment of pulmonary fibrosis.

### Example 2:

Effects of annexin A5 on cell morphology of silica-induced human type II lung epithelial cells:
It is generally believed that the development and progression of IPF are induced by epithelial-driven micro-damage. Micro-damage to alveolar epithelial cells ultimately leads to myofibroblast activation. For idiopathic pulmonary fibrosis, silica-induced pulmonary fibrosis was studied *in vitro*, which acted on type II alveolar epithelial cells, and the morphology and degree of damage repair of the alveolar epithelium were primarily compared.

Experimental methods: human primary type II alveolar epithelial cells (Wuhan Saios Biotechnology Co., Ltd.) were seeded into 6-well plates at a density of 2 × 10⁵ cells/well and cultured in primary epithelial cell culture medium containing 2% FBS (Wuhan Saios Biotechnology Co., Ltd.) at 37 °C for 24 hours. After starving the cells in primary epithelial cell culture medium containing 0.5% BSA for 8 hours, silica suspension (dissolved in PBS) was added to each well with 100 µg/ml. The cells were then treated with different concentrations of annexin A5: 10 µg/ml and 200 µg/ml, and cultured for another 24 hours. After culture, the effects on morphology of type II alveolar epithelial cells was observed under a microscope: the differences in morphology among the blank control group, the model control group, and the experimental groups were compared. After washing the cells in each group twice with PBS, the changes in the expression of the epithelial phenotypic marker - lung surfactant protein C (SFTPC) at the mRNA level were detected according to the instructions of the ordinary cell RNA extraction kit and fluorescence quantitative PCR detection kit.

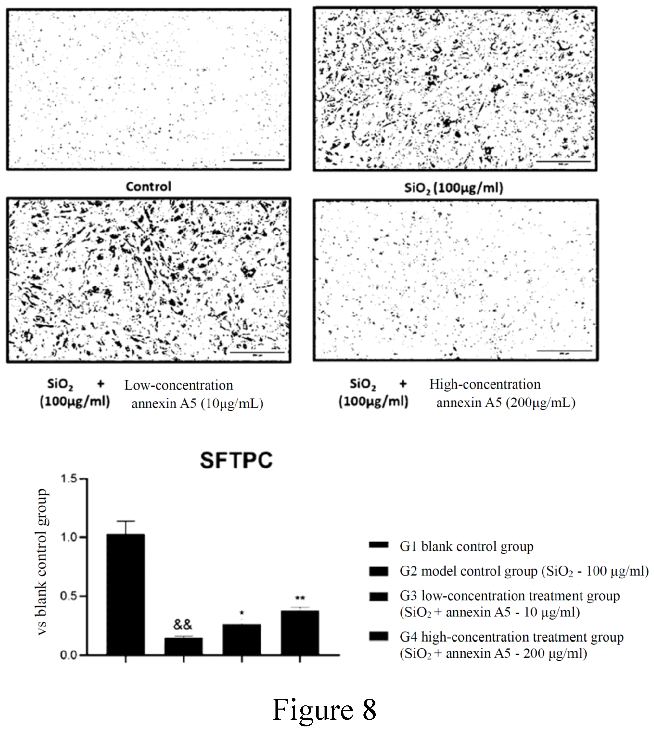

The results showed that, compared with the blank control group, the human alveolar type II epithelial cells in the model control group underwent significant morphological transformation after treatment with silica suspension, changing from cuboidal to elongated polygonal shapes, indicating epithelial-mesenchymal transition in damaged epithelial cells (Figure 8). The addition of high-concentration annexin A5 (200 µg/ml) effectively maintained the epithelial morphology of the cells and had a protective effect against silica-induced morphological transformation of type II epithelial cells (Figure 8). After treatment with silica suspension, compared with the blank control group, the expression of the type II epithelial cell phenotypic marker SFTPC of the model control group was reduced at the mRNA level (*p* < 0.01) (Figure 8). The addition of both low and high concentrations of annexin A5 remodeled the expression of SFTPC at the mRNA level (*p* < 0.05, *p* < 0.01), and the treatment effect of high concentration was better. Therefore, annexin A5 has a protective effect against silica-induced phenotype of type II epithelial cells (Figure 8).

All documents mentioned in the present invention are incorporated herein by reference as if each document were individually incorporated by reference. Furthermore, it should be understood that after reading the foregoing teachings of the present invention, those skilled in the art can make various changes or modifications to the present invention, and these equivalent forms also fall within the scope defined by the appended claims.

## Claims

1. Use of annexin A5 in the preparation of a composition for the prevention and/or treatment of idiopathic pulmonary fibrosis.

2. The use according to claim 1, wherein the idiopathic pulmonary fibrosis is an idiopathic pulmonary fibrosis induced by one or more factors selected from the group consisting of: infection, environmental exposure, smoking aging, genetics, and gene mutations.

3. The use according to claim 1, wherein the idiopathic pulmonary fibrosis is an idiopathic pulmonary fibrosis induced by micro-damage to alveolar epithelial cells.

4. The use according to claim 1, wherein the idiopathic pulmonary fibrosis is an idiopathic pulmonary fibrosis induced by drug treatment.

5. The use according to claim 1, wherein the prevention and/or treatment of idiopathic pulmonary fibrosis comprises one or more features selected from the group consisting of:
(1) improvement of lung function or maintenance of normal lung physiological function;
(2) reduction of inflammatory cell infiltration, wherein the inflammatory cell infiltration occurs in one or more of the following sites: bronchus, bronchioles, terminal bronchioles, respiratory bronchus, alveolar ducts, alveolar sacs, alveoli, or pulmonary interstitium, or perivascular regions thereof;
(3) reduction of fibrosis, wherein the fibrosis occurs in one or more of the following sites: bronchi, bronchioles, terminal bronchioles, respiratory bronchioles, or pulmonary interstitium, or perivascular regions thereof;
(4) reduction of damage to bronchioles and pulmonary arterioles within the lesions and at the margins of ;
(5) inhibition of the expression of profibrotic biomarkers;
(6) inhibition of the release of pro-inflammatory factors, wherein the release of pro-inflammatory factors occurs in one or more of the following sites: alveolar bronchial lavage fluid or lung tissue homogenate;
(7) inhibition of the differentiation of fibroblasts;
(8) inhibition of deposition of collagen in lung tissue;
(9) maintenance of the morphology of alveolar epithelial cells in lung tissue;
(10) maintenance of the expression of alveolar epithelial phenotypic markers in lung tissue;
(11) inhibition of the expression of profibrotic factors in lung tissue.

6. The use according to claim 5, wherein the damage to bronchiolars and pulmonary arterioles comprises one or more features selected from the group consisting of:
(1) reduction of alveolar hemorrhage;
(2) reduction of the lung epithelial cell proliferation, wherein the epithelial cell proliferation occurs in one or more of the following sites: the bronchioles and terminal bronchioles;
(3) reduction of the proliferation of granulation tissue on the adventitia of the tube wall, wherein the granulation tissue on the adventitia of the tube wall occurs in one or more of the following sites: bronchioles and terminal bronchioles;
(4) reduction of inflammatory cell infiltration, wherein the inflammatory cell infiltration occurs in one or more of the following sites: the bronchus, bronchioles, terminal bronchioles, respiratory bronchioles, alveolar ducts, alveolar sacs, alveoli, pulmonary interstitium, or pulmonary arterioles, or perivascular regions thereof;
(5) reduction of edema, wherein the edema occurs in one or more of the following sites: the bronchi, bronchioles, terminal bronchioles, or respiratory bronchioles, or perivascular regions thereof;
(6) reduction of endothelial cell shedding, wherein the endothelial cell shedding occurs in pulmonary arterioles.

7. The use according to claim 1, wherein the composition further comprises a pharmaceutically acceptable carrier.

8. The use according to claim 1, wherein the dosage form of the composition includes tablet, lozenge, powder, granule, capsule, injection, tincture, oral liquid, powder inhaler, aerosol, or spray.

9. The use according to claim 1, wherein the composition is administered by injection, nebulized inhalation, or oral administration.

10. The use according to claim 1, wherein the composition further comprises an additional drug for preventing and/or treating idiopathic pulmonary fibrosis.
